# EUROPEAN PATENT APPLICATION

(11) **EP 2 700 640 A1**
(43) Date of publication of application: **26.02.2014**
(21) Application number: 12181397.6
(22) Date of filing: 22.08.2012
(51) Int. Cl.: C07D 487/22, H01G 9/20, H01L 51/00

(54) **Azulenocyanine compounds, method of making the same, and their use as semiconductor and absorber for organic photovoltaics**

(71) Applicant: Solvay SA, 1120 Bruxelles (BE)
(72) Inventor: Braun, Max Josef, 309000 Wedemark (DE); Gutmann, Sebastian, 30625 Hannover (DE); Woehrle, Dieter, 28359 Bremen (DE); Miyaji, Taichi, Zama-shi, Kanagawa-ken, Kanagawa 252-0004 (JP)
(74) Representative: Mross, Stefan P.M.

(57) **Abstract**

Disclosed are azulenocyanine compounds, method of making the same, and their use as semiconductor and absorber for organic photovoltaics. The azulenocyanine compounds according to the present invention have the following formula (I) or its regioisomers : 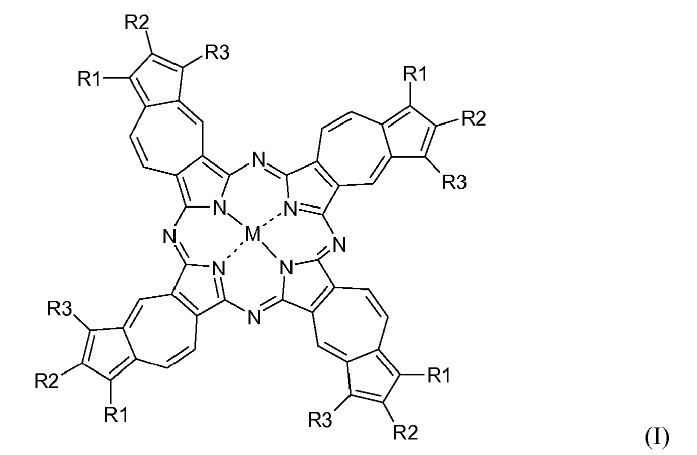 wherein M is selected from the group consisting of 2H (metal free analogue), metal cations in the oxidation state +2, preferably Zn(II), Mg(II), Cd(II), Cu(II), Ni(II), cations in the oxidation state >+2, preferably Ti(IV)O, V(IV)O, Al(III)X, Si(IV)X₂, Sn(IV)X₂, and Ge(IV)X₂, wherein X is selected from halides, OH and ORa with Ra being alkyl groups or aryl groups ; and at least one of R1, R2 and R3 is selected from the group consisting of -CH₂-R", -N=N-R', -COH(R')₂, -CH=CH-R"', and -C≡C-R"'.

## Description

### TECHNICAL FIELD

The present invention relates to azulenocyanine compounds, method(s) of making them, and their use as absorbers in photoelectric conversion devices, in particular as semiconductor and absorber in solid-state organic photovoltaics (OPV), and as additional dye in dye-sensitized solar cells (DSSC).

### BACKGROUND OF THE INVENTION

Conventional solar cells convert light into electricity by exploiting the photovoltaic effect that exists at semiconductor junctions. In other words, the commercial solar cells absorb energy from visible light and convert excited charge carriers thereof to electric energy. At present, the main commercial solar cells are silicon-based solar cells. For the silicon-based solar cell, there are shortcomings in that high energy costs for material processing is required and many problems to be addressed such as environmental burdens and cost and material supply limitations are involved. For an amorphous silicon solar cell, there are also shortcomings in that energy conversion efficiency decreases when used for a long time, due to deterioration in a short period.

One of the low-cost organic solar cells is a dye-sensitized solar cell (DSSC) which is based on a semiconductor formed based on a semiconductor sensitized electrode, a counter electrode and an electrolyte, and generally uses an organic dye to absorb incoming light to produce excited electrons. Macrocyclic compounds, one of the organic dyes used in this connection, especially porphyrin derivatives and recently also phthalocyanine derivatives are subject of interest for this type of sensitization dyes in DSSC. They can exhibit conversion efficiencies up to around 12 % and 5.5 %, respectively (A. Yella, Md. Nazeeruddin, M. Grätzel et al, Science 2011, 334, 629-634 ; M-E. Ragoussi, Md. Nazeeruddin, M. Grätzel et al, Angew. Chem. Int. Ed. 2012, 51, 4375-4378).

Another low-cost organic solar cell is a solid-state organic photovoltaic comprising thin films of electron donor and electron acceptor organic materials, and in addition, optional electron/hole transporting and exciton blocking layers. Among the electron donor organic materials are dyes having phthalocyanine structure.

Japanese Patent Application Kokai JP 2011-116717A discloses a compound which is a condensation product of certain azulene rings having a formula (1) below and its isomer, which absorbs a near-infrared-light : 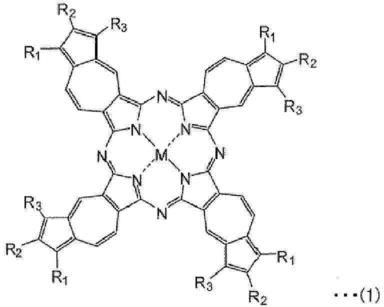 wherein R1, R2 and R3 are independently a hydrogen atom, an aromatic ring group which may have a substituent, or a non-aromatic ring substituent having 0-20 carbon, where the R1 and R2, R2 and R3 existing in the identical azulene ring may form a ring unitedly, respectively ; and M is two hydrogen atoms or the cation atom is at least bivalent ; in case said cation atom is at least trivalent, M may further comprise a counter-anion having no more than 20 carbons.

### DESCRIPTION OF THE INVENTION

The purpose of the present invention is to provide novel azulenocyanine compounds having advantageous performance(s) when used in photoelectric conversion devices, especially in dye sensitized solar cells (DSSC) or in solid-state organic photovoltaics (OPV).

The present invention therefore relates to azulenocyanine compounds, having the following formula (I) or its regioisomers : 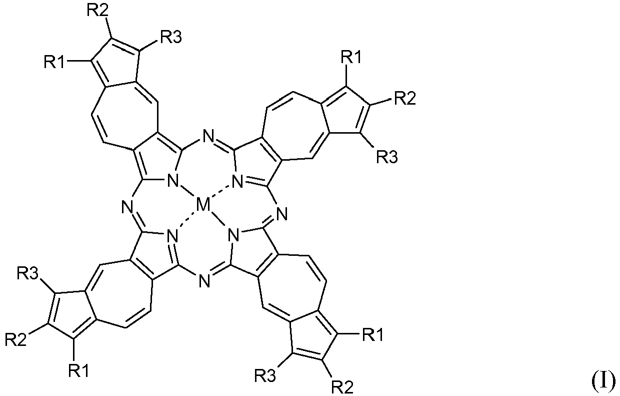 wherein : M is selected from the group consisting of 2H (metal free analogue), metal cations in the oxidation state +2, preferably Zn(II), Mg(II), Cd(II), Cu(II), and Ni(II), cations in the oxidation state >+2, preferably Ti(IV)O, V(IV)O, Al(III)X, Si(IV)X₂, Sn(IV)X₂, and Ge(IV)X₂, wherein X is selected from halides, OH and ORa with Ra being an alkyl or aryl group ; and R1, R2 and R3 are independently selected from the group consisting of hydrogen, hydroxyl, nitro, cyano, halogens, alkyl groups, halogenated alkyl groups, alkoxy groups, halogenated alkoxy groups, alkylthio groups, aryl groups, halogenated aryl groups, aryloxy groups, halogenated aryloxy groups, arylthio groups, -OC-R' (wherein R' is selected from hydrogen, alkyl groups, halogenated alkyl groups, aryl groups, and halogenated aryl groups), -CH₂-R" (wherein R" is selected from the group consisting of alkoxy groups, halogenated alkoxy groups, aryloxy groups and halogenated aryloxy groups and -NR'₂), -N=N-R', -COH(R')₂, -CH=CH-R', -S-R' and -C≡C-R' provided at least one of R1, R2 and R3 is selected from the group consisting of -CH₂-R", -N=N-R', -COH(R')₂, -CH=CH-R"', and -C≡C-R"' (wherein R"' is selected from aryl groups, halogenated aryl groups and halogenated alkyl groups).

It has been surprisingly found that the azulenocyanine compounds according to the present invention provide a wider range of light absorption when used in photoelectric conversion devices, in particular as semiconductor and absorber in solid-state OPV. Also, the azulenocyanine compounds according to the present invention can be used as additional absorber in dye-sensitized solar cells (DSSC).

The azulenocyanine compounds according to the present invention, when used especially in solid-state OPV, can function, in dependence of electron-donating or -withdrawing substituents, as effective electron donor or electron acceptor material which absorbs the wide-range of solar radiation including the whole visible region and near-IR regions, noting that about 48 % of the solar radiation is in the visible region and about 52 % in the near-IR region.

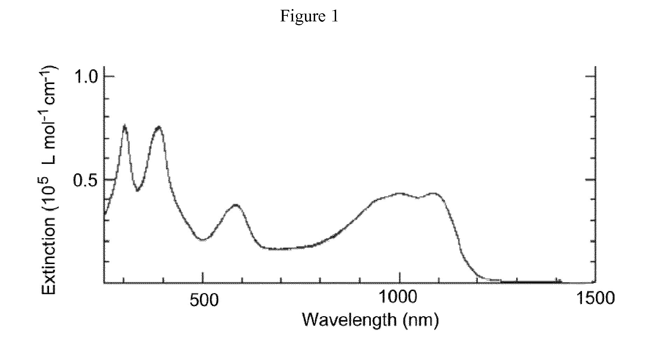

It has also been surprisingly found that the azulenocyanine compounds according to the present invention provide improved characteristics, for example absorptions from the visible region of light to the NIR region (Figure 1), a high molar extinction coefficient and therefore high conversion efficiencies. In addition, it has been found that a wider-range of light absorption when used in photoelectric conversion devices, such as in solid-state OPV and in DSSC, can be attained by the azulenocyanine compounds according to the present invention. In the present invention, "azulene" is understood to denote in particular an organic compound comprising a structure below :

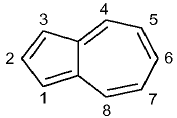

In the present invention, "alkyl groups" is understood to denote in particular a straight chain, branched chain, or cyclic hydrocarbon groups usually having from 1 to 20 carbon atoms, preferably having from 1 to 8 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, "alkoxy groups" is understood to denote in particular a straight chain, branched chain, or cyclic hydrocarbon groups usually having from 1 to 20 carbon atoms, preferably having from 1 to 8 carbon atoms, singularly bonded to oxygen (Alk-O-).

In the present invention, "alkylthio groups" is understood to denote in particular a straight chain, branched chain, or cyclic hydrocarbon groups usually having from 1 to 20 carbon atoms, preferably having from 1 to 8 carbon atoms, singularly bonded to sulfur (Alk-S-).

In the present invention, "aryl groups" is understood to denote in particular any functional group or substituent derived from an aromatic ring. In particular, the aryl groups can have 6 to 20 carbon atoms preferably 6 to 12 carbon atoms, in which some or all of the hydrogen atoms of the aryl group may or may not be substituted with other groups, especially alkyl groups, alkoxy groups, thioalkyl groups, aryl groups, hydroxyl groups or halogens such as fluoro. The aryl groups are preferably optionally substituted phenyl groups, naphthyl groups, anthryl group and phenanthryl group.

In the present invention, "aryloxy groups" is understood to denote in particular the aryl group as defined above singularly bonded to oxygen (Ar-O-).

In the present invention, "arylthio groups" is understood to denote in particular the aryl group as defined above singularly bonded to sulfur (Ar-S-).

In the present invention, "heterocycles" is understood to denote in particular a cyclic compound which has at least one heteroatom as a member of its one or more rings. Frequently, heteroatoms within the ring include sulfur, oxygen and nitrogen. The heterocycles can be either saturated or unsaturated, and may be a 3-membered, 4-membered, 5-membered, 6-membered or 7-membered ring. The heterocycles can be further fused with other one or more ring systems. Examples of the heterocycles include pyrrolidines, oxolanes, thiolanes, pyrroles, furans, thiophenes, piperidines, oxanes, thianes, pyridines, quinolines, pyrans, and thiopyrans, and their derivatives. A particular class of the heterocycles includes substituents comprising thiophene moiety. The heterocycles can further be substituted by other groups, such as alkyl groups, alkoxy groups, aryl groups or aryloxy groups as defined above.

In the present invention, "halogenated" is understood to denote in particular at least one of the hydrogen atoms of the respective chemical group has been replaced by a halogen atom, preferably selected from fluorine and chlorine, more preferably fluorine. If all of the hydrogen atoms have been replaced by halogen atoms, the halogenated chemical group is perhalogenated. For instance, "halogenated alkyl groups" include (per)fluorinated alkyl groups such as (per)fluorinated methyl, ethyl, propyl, isopropyl, butyl, isobutyl, *sec*-butyl or *tert*-butyl; and for instance -CF₃, -C₂F₅, heptafluoroisopropyl (-CF(CF₃)₂), hexafluoroisopropyl (-CH(CF₃)₂) or perfluoro-n-hexyl [-CF₂(CF₂)₄CF₃]. Non-limiting example of "halogenated aryl groups" is -C₆F₅.

In the present invention, the regioisomers of the formula (I) includes the structures below : 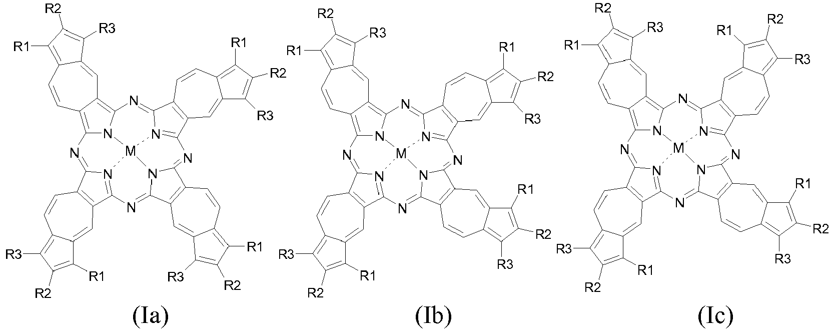 wherein M, R1, R2 and R3 have the same meaning as defined above.

In one embodiment, R1 and R3 are the same and are selected from the group consisting of -CH₂-R", -N=N-R', -COH(R')₂, -CH=CH-R"', and -C≡C-R"'. In this embodiment, R2 is preferably hydrogen.

In another embodiment, the azulenocyanine compounds according to the present invention comprise, for example, at least one fluorine atom. Preferably, substituents R1, R2 and/or R3 comprise at least one fluorine atom, more preferably, R1 and R3 comprise at least one fluorine atom.

The azulenocyanine compounds according to the present invention described herein are suitable for use in photoelectric conversion devices, particularly in solid-state organic photovoltaics (OPV) or in dye-sensitized solar cells (DSSC).

The present invention concerns the use of the azulenocyanine compounds according to the present invention as semiconductor and absorber suitable for use in solid-state organic photovoltaic (OPV). Further, the present invention also relates to the use of the azulenocyanine compounds according to the present invention as an additional absorber suitable for use in dye-sensitized solar cells (DSSC).

Still further, the present invention concerns a method of production of photoelectric conversion devices, in particular in solid-state organic photovoltaics (OPV) or in dye-sensitized solar cells (DSSC), wherein the azulenocyanine compound according to the present invention is used.

The solid-state OPV provides the prospect of a cheap and versatile technology for large scale production of solar cells. When the light absorbed by electron donor material, the excited state which can be regarded as electron-hole pair bound together by electrostatic interactions (exciton) is formed. In solid-state OPV cells, excitons are broken up into free electron-hole pairs by effective fields. The same is valid for an electron acceptor material. The effective fields are set up by creating a heterojunction between two dissimilar materials. Effective fields break up excitons by causing the electron to fall from the conduction band of the absorber to the conduction band of the acceptor molecule.

The basic element of a solid-state OPV is one or more of organic layer(s) comprising organic material(s) disposed between the two electrodes. In addition, further optional one or more layer(s), for instance, electron transporting layer, hole transporting layer and/or exciton blocking layer, can be fabricated to optimize the solid-state OPV device. The layer for the electron donor and electron acceptor materials can be formed as separate two layers, or as one mixed heterojunction layer.

In this connection, the azulenocyanine compound according to the present invention can be used as an organic material, in particular as an electron donor material or an electron acceptor material in dependence of the kind of substituents at the azulenocyanine compounds, in such device or cell. Accordingly, the present invention further relates to a photoelectric conversion device, preferably a solid-state organic photovoltaic (OPV), which comprises the azulenocyanine compound of the present invention used as semiconductor and absorber.

OPV cells with the azulenocyanine compounds according to the present invention are obtained either by vapor deposition employing, for examples, azulenocyanine compounds or fluorinated azulenocyanine compounds, or from solution by printing or spin coating using the azulenocyanine compounds with different above mentioned substituents R.

The DSSC offers the prospect of a cheap and versatile technology for large scale production of solar cells. The dye-sensitized solar cell (DSSC) is formed by a combination of organic and inorganic components that could be produced at a low cost. The dye-sensitized solar cells have advantages over silicon-based solar cells in terms of simplified processing steps, low fabrication cost, transparency and pleochroism. The dye-sensitized solar cells can be fabricated from flexible substrates to function as cells of mobility and portability. Dye-sensitized solar cells have also the advantage to be lightweight.

The dye-sensitized solar cells have lower energy (photoelectric) conversion efficiency over that of the silicon-based solar cells such that a wide range of researches are briskly under way to enhance the energy conversion efficiency. In order to improve the energy conversion efficiency, extension of wave length up to infrared regions is being waged with great concern. The energy bandgap (eV) for use in solar cells must exceed 1.4 eV (electron volt).

The basic element of a DSSC is generally a TiO₂ (titanium dioxide) nanoparticulate structure sensitized with dye molecules to form the core of a DSSC. The assembly of titanium dioxide nanoparticles is well connected to their neighbors. TiO₂ is the preferred material for the nanoparticles since its surface is highly resistant to the continued electron transfer. However, TiO₂ only absorbs a small fraction of the solar photons (those in the UV). The dye molecules attached to the semiconductor surface are used to harvest a great portion of the solar light.

The azulenocyanine compounds according to the present invention do not contain an anchoring group for binding at semiconductor materials, such as TiO₂ or TiOF₂, as the conventionally employed dyes in DSSC. Therefore, the azulenocyanine compounds of the present invention are used as additional absorber in such devices or cells. The azulenocyanine compound with substituents R1, R2 and R3 as mentioned above can be deposited by printing, spin coating or dip coating for example onto a TiO₂/dye electrode. Accordingly, the present invention further relates to a photoelectric conversion device, preferably a dye-sensitized solar cell (DSSC), which comprises the azulenocyanine compound of the present invention as additional absorber.

The present invention relates to a method for making the above-described azulenocyanine compounds. The method comprises a step of reacting, preferably condensing, at least one 5,6-dicyanoazulene comprising at least one substituent selected from the groups -CH₂-R", -N=N-R', -COH(R')₂, -CH=CH-R"', and -C≡C-R"', as building blocks for the macrocyclic structure.

Said reaction can be performed by condensing statistically four molecules of the at least one 5,6-dicyanoazulene. The condensation can be optionally performed in a presence of catalyst and/or solvent.

The examples of the 5,6-dicyanoazulene for said method include the following structures : 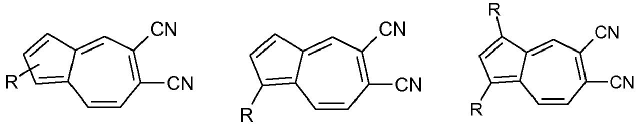 wherein R has the same meaning for R1, R2 and R3 as mentioned above.

In the present invention, said 5,6-dicyanoazulene can be synthesized by electrophilic aromatic substitution by the electrophile E⁺ in the 1- or 1,3-positions of the azulene ring, as exemplified below :

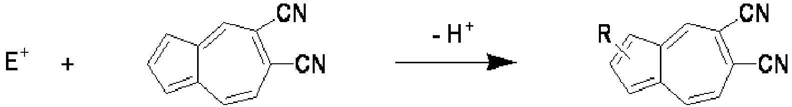

In a further embodiment, the mechanism for the electrophilic aromatic substitution is selected from chloromethylation, diazo coupling reaction, hydroxyalkylation or Suzuki coupling / Heck coupling / Stille coupling reaction.

In case of chloromethylation, Cl-CH₂ substituted azulene dinitrile is first prepared. Then, as one way, subsequent reactions with non-fluorinated and fluorinated alcohols, phenols and heterocycles to form -CH₂-R" substituted azulene dinitriles, with a preference for R" being alkoxy groups, aryloxy groups, fluorinated alkoxy groups or fluorinated aryloxy groups. The another way is to subsequently react the -Cl-CH₂ substituted azulene dinitrile with non-fluorinated and fluorinated aliphatic and aromatic/heterocyclic amines to for -CH₂-R" substituted azulene dinitriles, with a preference for R" being -NR'₂ (wherein R' is alkyl groups, aryl groups, fluorinated alkyl groups or fluorinated aryl groups).

In case of diazo coupling, reaction with diazonium salts can be performed to result in -N=N-R', with a preference for R' being aryl groups or fluorinated aryl groups.

In case of hydroxyalkylation, -COH(R')₂ substituted 5,6-dicyanoazulene, with a preference for R' being alkyl groups, aryl groups, or fluorinated alkyl groups or fluorinated aryl groups.

In case of Suzuki coupling / Heck coupling / Stille coupling reaction, first 5,6-dicyanoazulene halide can be first converted to R"' substituted 5,6-dicyanoazulene, and then converted to -CH=CH-R"' substituted 5,6-dicyanoazulene, with a preference for R"' being aryl groups, fluorinated alkyl groups or fluorinated aryl groups.

In case of ethynyl bonds, iodoazulenes obtained from 5,6-dicyanoazulene with *N*-iodosuccinimide are reacted with an alkylacetylene, arylacetylene or trimethylsilylacetylene in the presence of a Pd catalyst and CuI to obtain 5,6-dicyanoazulenes with R-≡-R"" (wherein R"" is selected from alkyl groups, aryl groups and trimethylsilyl group). The trimethylsilyl group can be splitted to obtain R-C≡-H.

In a particular embodiment, the method for making the azulenocyanine compounds according to the present invention further comprises a step of utilizing an M-containing precursor. The M-containing precursor is preferably a metal salt or derivative of M more preferably a Zn salt such as zinc acetate, magnesium oxide, an aluminum derivative such as aluminum chloride or bromide, a silicon derivative such as tetrachlorosilane, a stannous halide (SnHal₂), or a tin derivative such as tin dichloride (SnCl₂).

The azulenocyanine compounds according to the present invention may be further isolated, for instance by column chromatography, preferably by high pressure liquid chromatography (HPLC).

While preferred embodiments of this invention have been shown and described, modifications thereof can be made by one skilled in the art without departing from the spirit or teaching of this invention. The embodiments described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

Should the disclosure of any patents, patent applications, and publications which are incorporated herein by reference conflict with the description of the present application to the extent that it may render a term unclear, the present description shall take precedence.

### Brief Description of the Drawing

### Figure 1 shows the electronic absorption spectrum of an azulenocyanine compound (mixture of regioisomers).

### Examples

### Example 1 : Preparation of zinc(II) 1,1',1",1"'-tetra[2-(phenyl)ethenyl]-azulenocyanine as mixture of regioisomers

To a stirred solution of 30 mL of dry 1-hexanol under inert gas 8 mmol of Li shot is added, and the reaction mixture is allowed to warm up to 100°C and stirred. After the Li metal is consumed, 1.5 mmol of 1-[2-(phenyl)ethenyl]-azulene is added, and the reaction mixture is warmed up to reflux for 8 h. Then, the reaction mixture is cooled and poured into MeOH and filtrated. The metal-free azulenocyanine compound is separated by silica gel column chromatography and then metalated with an ecess of Zn(OAc)₂ in DMF. The black coloured zinc(II) complex is obtained in a yields of 47 %.

### Example 2 : Preparation of zinc(II) 1,1',1",1"'-tetra(azobenzene)-azulenocyanine as mixture of regioisomers

6 mmol of azuleno-1-azobenzol and 2.1 mmol of dry Zn(OAc)₂ are suspended in 60 mL dry 1-pentanol. The mixture is heated under inert gas and stirring at 145°C. 3 mmol of DBU is added, and heating is continued for 24 h at 145°C. The cooled reaction mixture is added to methanol, and the precipitate is filtered. Then, the azulenocyanine compound is isolated by column chromatography on silica gel. The black coloured azulenocyanine compound is obtained in a yield 51 %.

## Claims

1. An azulenocyanine compound, having the following formula (I) : 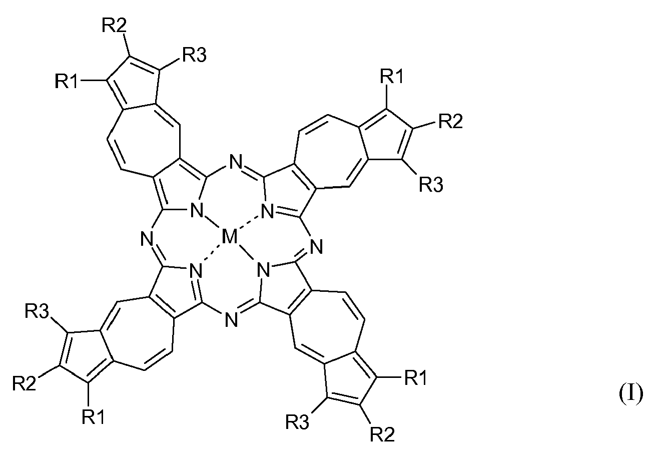 wherein :
M is selected from the group consisting of 2H (metal free analogue), metal cations in the oxidation state +2, preferably Zn(II), Mg(II), Cd(II), Cu(II), Ni(II), cations in the oxidation state >+2, preferably Ti(IV)O, V(IV)O, Al(III)X, Si(IV)X₂, Sn(IV)X₂, and Ge(IV)X₂, wherein X is selected from halides, OH and ORa with Ra being an alkyl groups or aryl groups ; and
R1, R2 and R3 is independently selected from the group consisting of hydrogen, hydroxyl, nitro, cyano, halogens, alkyl groups, halogenated alkyl groups, alkoxy groups, halogenated alkoxy groups, alkylthio groups, aryl groups, halogenated aryl groups, aryloxy groups, halogenated aryloxy groups, arylthio groups, -OC-R' (wherein R' is selected from hydrogen, alkyl groups, halogenated alkyl groups, aryl groups, and halogenated aryl groups), -CH₂-R" (wherein R" is selected from the group consisting of alkoxy groups, halogenated alkoxy groups, aryloxy groups and halogenated aryloxy groups and -NR'₂), -N=N-R', -COH(R')₂, -CH=CH-R', -S-R' and -C≡C-R' provided at least one of R1, R2 and R3 is selected from the group consisting of -CH₂-R", -N=N-R', -COH(R')₂, -CH=CH-R"', and -C≡C-R"' (wherein R"' is selected from aryl groups, halogenated aryl groups and halogenated alkyl groups),
or its regioisomers.

2. The azulenocyanine compound according to Claim 1, wherein R1 and R3 are the same and selected from the group consisting of -CH₂-R", -N=N-R', -COH(R')₂, -CH=CH-R"', and -C≡C-R"', and R2 is hydrogen.

3. The azulenocyanine compound according to Claim 1 or 2, comprising at least one fluorine atom, preferably substituents R1, R2 and/or R3 comprise at least one fluorine atom.

4. Use of the azulenocyanine compound according to any one of Claims 1 to 3, as a semiconductor and/or as an absorber suitable for use in solid-state organic photovoltaic device (OPV).

5. Use of the azulenocyanine compound according to any one of Claims 1 to 3, as an absorber suitable for use in dye-sensitized solar cell (DSSC).

6. A method for making the azulenocyanine compound according to any one of Claims 1 to 3, comprising a step of reacting, preferably condensing, at least one 5,6-dicyanoazulene comprising at least one substituent selected from the groups -CH₂-R", -N=N-R', -COH(R')₂, -CH=CH-R"', and -C≡C-R"'.

7. The method according to Claim 6, further comprising a step of utilizing an M-containing precursor, preferably a metal salt or derivative of M more preferably a Zn salt such as zinc acetate, magnesium oxide, an aluminum derivative such as aluminum chloride or bromide, a silicon derivative such as tetrachlorosilane, a stannous halide (SnHal₂), or a tin derivative such as tin dichloride (SnCl₂).

8. The method according to Claim 6 or 7, wherein the 5,6-dicyanoazulene comprising at least one substituent is synthesized by electrophilic aromatic substitution, preferably by chloromethylation, diazo coupling reaction, hydroxyalkylation, Suzuki coupling reaction, Heck coupling reaction or Stille coupling reaction.

9. A solid-state organic photovoltaic device (OPV) comprising the azulenocyanine compound according to any one of Claims 1 to 3.

10. A dye sensitized solar cell device (DSSC) comprising the azulenocyanine compound according to any one of Claims 1 to 3.
